## Europäisches Patentamt

## European Patent Office

## Office européen des brevets

(19)

(11) Veröffentlichungsnummer: **0 191 122**
**B1**

(12) # EUROPÄISCHE PATENTSCHRIFT

(45) Veröffentlichungstag der Patentschrift:
**23.11.88**

(51) Int. Cl.⁴: **A 61 M 5/34**

(21) Anmeldenummer: **85101508.1**

(22) Anmeldetag: **13.02.85**

(54) **Spritze für medizinische Zwecke.**

(43) Veröffentlichungstag der Anmeldung:
**20.08.86 Patentblatt 86/34**

(45) Bekanntmachung des Hinweises auf die Patenterteilung:
**23.11.88 Patentblatt 88/47**

(84) Benannte Vertragsstaaten:
**AT BE CH DE FR GB IT LI LU NL SE**

(56) Entgegenhaltungen:
**US-A-2 755 801**
**US-A-3 096 763**

(73) Patentinhaber: **Arzneimittel GmbH Apotheker Vetter & Co. Ravensburg, Marienplatz 79- 81, D-7980 Ravensburg (DE)**

(72) Erfinder: **Geprägs, Peter, Scherzachstrasse 5, D-7981 Schlier (DE)**

(74) Vertreter: **Fay, Hermann, Dipl.- Phys. Dr., Ensingerstrasse 21 Postfach 1767, D-7900 Ulm (Donau) (DE)**

EP 0 191 122 B1

**Beschreibung**

Die Erfindung betrifft eine Spritze für medizinische Zwecke mit einem einen Anschlußhals zum Ansatz der Spritzennadel bildenden, am Zylindermundstück des Spritzenzylinders aufgesetzten Nadelansatzstück, und mit einer das Nadelansatzstück am Spritzenzylinder haltenden, am Zylindermundstück eingerasteten Überwurfkappe, die auf ihrer dem Zylindermundstück abgewandten Seite einen Kragen aufweist, der den Anschlußhals außen dichtschließend ummantelt und der aus einem im Vergleich zu dem gummielastischen Werkstoff des Nadelansatzstückes elastisch wesentlich steiferen Kunststoff besteht und dadurch den Anschlußhals mechanisch versteift.

Spritzen dieser Gestaltung ohne allerdings den Kragen sind in der nicht vorveröffentlichten, zum Stand der Technik nach EPÜ Art. 54 (3), (4) gehörenden EP-A-0 144 483 (Anmelde-Nr. 84 100 711.5) beschrieben und haben den Vorteil, daß das Nadelansatzstück und die Überwurfkappe auch bei schon befüllter Spritze leicht montiert werden können und einen sicheren Abschluß des Spritzen-Zylinders ergeben. Zur Montage genügt es, die Überwurfkappe über das dem Zylindermundstück aufgesetzte Nadelansatzstück zu stecken und bis zum Einrasten am Zylindermundstück festzudrücken. Die Spritzennadel kann in den aus der Überwurfkappe vorragenden Anschlußhals einvulkanisiert oder temperaturbeständig eingeklebt sein. Sie kann aber auch zunächst fehlen und erst unmittelbar vor der Applikation der Spritze in oder auf den Anschlußhals ein- bzw. aufgesteckt werden, wozu im letzteren Fall am Anschlußhals in üblicher Weise ein Luerkonus angeformt sein kann, auf den eine die Spritzennadel tragende Nadelmuffe paßt. Auch kann bei anders am Nadelansatzstück angebrachter Spritzennadel auf den Luerkonus eine die Spritzennadel aufnehmende und schützende Kappe aufgesteckt werden. Wird die Spritzennadel erst unmittelbar vor der Applikation der Spritze auf den Anschlußhals aufgesteckt, kann bis dahin der im Nadelansatzstück den Austritt des Injektionsmittels ermöglichende Kanal durch eine Verschlußkappe nach Art einer sogenannten Tip-Cap verschlossen sein, die auf dem Luerkonus sitzt und einen in den Kanal eingreifenden und ihn abdichtenden zentralen Stift besitzt.

Bei solchen Spritzen kann das Problem auftreten, daß das Nadelansatzstück in seinen elastischen Werkstoffeigenschaften einerseits möglichst weich eingestellt sein soll, damit es eine gute und zuverlässige Abdichtung am Zylindermundstück gewährleistet, dann aber andererseits im Bereich des Anschlußhalses nicht mehr genügend steif ist, um die Spritzennadel noch mit ausreichender mechanischer Stabilität halten zu können. Dieses Problem wird bei einer aus der US-A-3 096 763 bekannten Spritze der eingangs genannten Art mit Hilfe des Kragens gelöst, der den Anschlußhals mechanisch versteift. Der Kragen umschließt den Anschlußhals wie eine Mantelhülse und ist, da er aus dem Kunststoff der Überwurfkappe besteht, in sich so fest, daß er Biegeverformungen des Anschlußhalses verhindert und dadurch eine gute mechanische Stabilität des Nadelanschlusses am Nadelansatzstück gewährleistet. Die Spritzennadel ist bei der bekannten Spritze in den Anschlußhals einvulkanisiert bzw. eingeklebt.

Darüber hinaus ist aus der US-A-2 755 801 eine Spritze bekannt, bei der unabhängig von der Überwurfkappe ein metallischer Kragen als innerer Teil und zur Verstärkung des Anschlußhalses vorgesehen ist.

Der Erfindung liegt die Aufgabe zugrunde, eine Spritze der eingangs genannten Art so weiterzubilden, daß die Spritzennadel auch mit einer sie tragenden Nadelmuffe oder daß eine Tip-Cap außen auf den Kragen aufgesteckt werden kann und daß die auf den Kragen aufgesetzte Nadelmuffe oder Tip-Cap hinreichend abgedichtet ist.

Diese Aufgabe wird dadurch gelöst, daß der Anschlußhals am nadelseitigen Ende einen elastischen Ringbund bildet, der die stirnseitige Ringfläche des Kragens radial auswärts bis zur äußeren Mantelfläche des Kragens übergreift und eine Ringdichtung gegenüber einer auf den Kragen aufgesetzten, die Spritzennadel tragenden Nadelmuffe oder einer Tip-Cap bildet.

Vorzugsweise bildet der Kragen an seiner äußeren Mantelfläche einen Luerkonus.

Im folgenden wird die Erfindung an in der Zeichnung dargestellten Ausführungsbeispielen näher erläutert; es zeigen:

Fig. 1 eine erfindungsgemäßen Spritzen lediglich schematisch angedeuteten Spritzenkolben im Axialschnitt,

Fig. 2 das vordere Ende des Spritzenzylinders und des Nadelansatzstückes ohne Spritzenadel im nicht zusammengesetzten Zustand und in gegenüber Fig. 1 vergrößerter Darstellung,

Fig. 3 den Gegenstand der Fig. 2 in einer geänderten Ausführungsform.

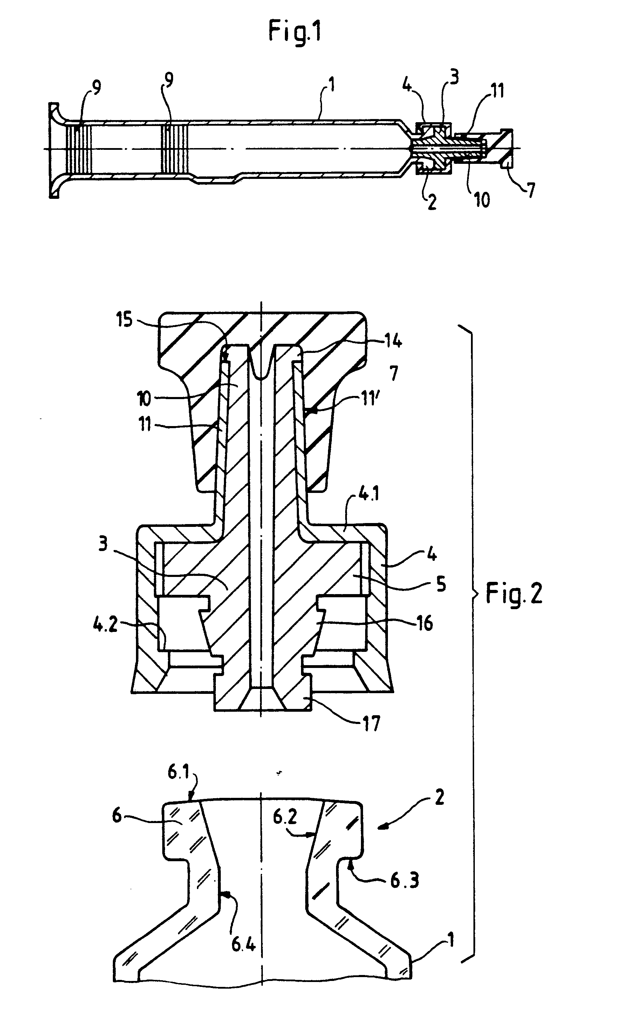

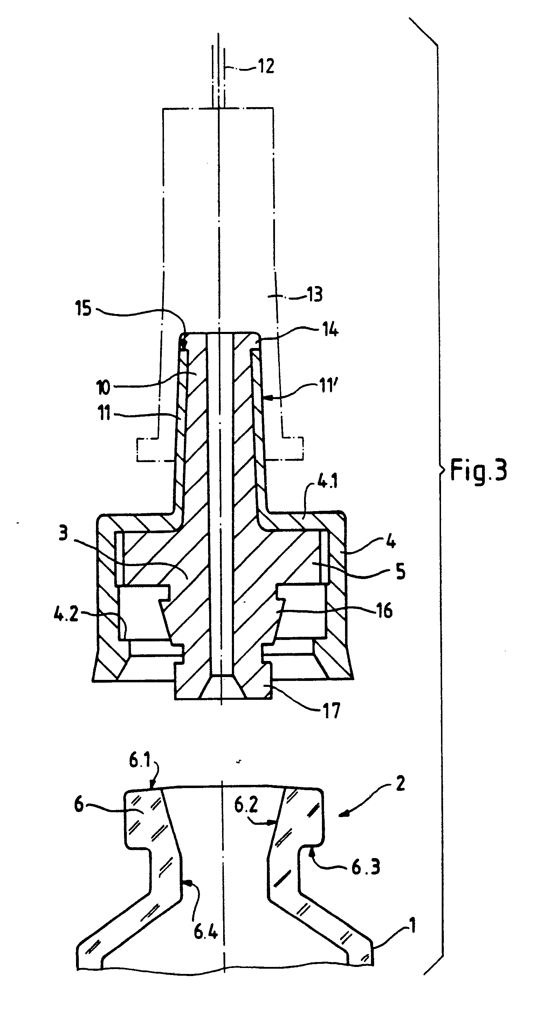

In der Zeichnung ist eine Zweikammerspritze mit zwei Spritzekolben 9 dargestellt und der Spritzenzylinder mit 1, sein Zylindermundstück mit 2 und das dem Zylindermundstück axial von außen aufsetzbare Nadelansatzstück mit 3 bezeichnet. Das Nadelansatzstück 3 wird durch eine Überwurfkappe 4 gehalten, wozu das Zylindermundstück 2 und das Nadelansatzstück 3 je einen Außenbund 5, 6 aufweisen und beide Außenbunde 5, 6 von der sie übergreifenden Überwurfkappe 4 axial gegeneinander verpreßt sind. Das Nadelansatzstück 3 ist zum Anschluß der Spritzennadel mit einem Anschlußhals 10 versehen. Die Überwurfkappe 4 bildet einen Kragen 11, der den Anschlußhals 10 außen dichtschließend ummantelt und dadurch versteift, wenn das Nadelansatzstück 3, um eine zuverlässige Abdichtung am Zylindermundstück 2 zu ergeben, aus einem vergleichsweise weich eingestellten gummielastischen Werkstoff und die Überwurfkappe 4 aus einem im Vergleich dazu elastich wesentlich steiferen Kunststoff besteht. Das Nadelansatzstück 3 kann, soweit an ihm noch keine Spritzennadel montiert ist, entsprechend den Fig. 1 und 2 durch ein Tip-Cap 7 verschlossen sein. Ist in das Nadelansatzstück 3 eine Spritzennadel eingesetzt, kann auf dem Anschlußhals 10 bzw. dem Kragen 11 eine in der Zeichnung nicht dargestellte Nadelschutzkappe aufgesteckt werden. Zum Nadelanschluß besteht weiter die in Fig. 3 dargestellte Möglichkeit, die Spritzennadel 12 mit einer sie tragen Nadelmuffe 13 außen über den Anschlußhals 10 und den Kragen 11 aufzustecken. In allen Fällen ist dabei der Kragen 11 an seiner äußeren Mantelfläche 11' in Form eines Luerkonus ausgebildet. In Fig. 2 bildet der Anschlußhals 10 am nadelseitigen Ende einen elastischen Ringbund 14, der die stirnseitige Ringfläche 15 des Kragens 11 radial auswärts bis zur äußeren Mantelfläche 11' des Kragens 11 übergreift und eine Ringdichtung gegenüber der konischen Innenfläche der auf den Kragen 11 aufgesetzten Nadelmuffe 13 oder Tip-Cap 7 bildet.

Das mit dem Spritzenzylinder 1 einstückige Zylindermundstück 2 besitzt eine sich nadelseitig erweiternde und bis zur nadelseitigen Stirnfläche 6.1 seines Außenbundes 6 erstreckende konische Innenfläche 6.2. Das insbesondere aus Gummi bestehende Nadelansatzstück 3 besitzt einen Konusteil 16, der bei am Zylindermundstück 2 fertig montierten Nadelansatzstück 3 dessen konischer Innenfläche 6.2 anliegt. Die den Außenbund 5 des Nadelansatzstückes 3 mit einem Ringflansch 4.1 übergreifende Überwurfkappe 4 ist am Zylindermundstück 2 mit einer die zylinderseitige Stirnfläche 6.3 des Aussenbundes 6 übergreifenden Innenschulter 4.2 elastisch eingerastet. Im Zylindermundstück 2 schließt sich zylinderseitig an die konische Innenfläche 6.2 eine zylindrische Innenfläche 6.4 an, der am Nadelansatzstück 3 ein Zylinderteil 17 entspricht, das bei am Zylindermundstück 2 montiertem Nadelansatzstück 3 mit seiner zylindrischen Außenfläche an der zylindrischen Innenfläche 6.4 des Zylindermundstücks 2 anliegt.

## Patentansprüche

1. Spritze für medizinische Zwecke, mit einem einen Anschlußhals (10) zum Ansatz der Spritzennadel (12) bildenden, am Zylindermundstück (2) des Spritzenzylinders (1) aufgesetzten Nadelansatzstück (3) und mit einer das Nadelansatzstück (3) am Spritzenzylinder (1) haltenden, am Zylindermundstück (2) eingerasteten Überwurfkappe (4), die auf ihrer dem Zylindermundstück (2) abgewandten Seite einen Kragen (11) aufweist, der den Anschlußhals (10) außen dichtschließend ummantelt und der aus einem im Vergleich zu dem gummielastischen Werkstoff des Nadelansatzstückes (3) elastisch wesentlich steiferen Kunststoff besteht und dadurch den Anschlußhals (10) mechanisch versteift, dadurch gekennzeichnet, daß der Anschlußhals (10) am nadelseitigen Ende einen elastischen Ringbund (14) bildet, der die stirnseitige Ringfläche (15) des Kragens (11) radial auswärts bis zur äußeren Mantelfläche (11') des Kragens (11) übergreift und eine Ringdichtung gegenüber einer auf den Kragen (11) aufgesetzten, die Spritzennadel (12) tragenden Nadelmuffe (13) oder einer Tip-Cap (7) bildet.

2. Spritze nach Anspruch 1, dadurch gekennzeichnet, daß der Kragen (11) an seiner äußeren Mantelfläche (11') einen Luerkonus bildet.

## Claims

1. A syringe for medical purposes having a needle mounting piece (3) forming a connecting neck (10) for mounting the injection needle (12) on the cylinder neck (2) of the syringe cylinder (1) and having a covering cap (4) engaging on the cylinder neck (2) and holding the needle mounting piece (2) on the syringe cylinder (1), the covering cap (4) having, on its side facing away from the cylinder neck (2), a collar (11) which encases the connecting neck (10) on the outside so as to form a sealing-tight seal and which consists of a substantially less elastic plastics than the rubber elastic material of the needle mounting piece (3) so as mechanically to stiffen the connecting neck (10), characterised in that the connecting neck (10) forms an elastic annular collar (14) on the end towards the needle, the annular collar radially outwardly overlapping the annular face (15) of the collar (11) on the end face as far as the outer casing surface (11') of the collar (11) and forming an annular seal with respect to a needle sleeve (13) or tip cap (7), which carries the needle (12) and which is disposed on the collar (1).

2. A syringe according to claim 1, characterised in that the collar (11) forms a Luercone on its outer casing surface (11').

## Revendications

1. Seringue a usage médical, comportant un élément (3) formant embout de montage d'une aiguille, qui constitue un col de raccordement (10) permettant le montage de l'aiguille (12) de la seringue et est emmanché sur l'élément d'embouchure (2) du cylindre de la seringue, et un capuchon d'accouplement (4), qui maintient l'élément (3) formant embout de montage de l'aiguille sur le cylindre (1) de la seringue, est emmanché par encliquetage sur l'élément d'embouchure (2) du cylindre et comporte, sur son côté tourné à l'opposé de l'élément d'embouchure (2) du cylindre, un collet (11) qui entoure extérieurement, d'une manière étanche, le col de raccordement (10), est constitué par une matière plastique élastique, nettement plus rigide que le matériau élastique de l'élément (3) formant embout de montage de l'aiguille et rigidifie par conséquent du point de vue mécanique le col de raccordement (10), caractérisée en ce que le col de raccordement (10) forme, sur son extrémité tournée vers l'aiguille, un rebord annulaire élastique (14), qui s'engage par-dessus la surface annulaire frontale (15) du collet (11), en s'étendant extérieurement du point de vue radial jusqu'à la surface extérieure (11') de ce collet, et forme et établit une étanchéité annulaire par rapport à un manchon (13) de l'aiguille ou a un Tip-Cap (7), mis en place sur le collet (11) et portant l'aiguille (12) de la seringue.

2. Seringue selon la revendication 1, caractérisée en ce que le collet (11) forme, au niveau de sa surface enveloppe extérieure (11'), un cône de Luer.

0 191 122

## Fig.1

## Fig.2

1

Fig.3